# EUROPEAN PATENT APPLICATION

(11) **EP 3 138 780 A1**
(43) Date of publication of application: **08.03.2017**
(21) Application number: 15786668.2
(22) Date of filing: 26.02.2015
(51) Int. Cl.: B65B 55/08, A61L 2/08, B65B 55/04

(54) **ELECTRON BEAM STERILIZATION METHOD FOR BOTTLE CAP AND ELECTRON BEAM STERILIZATION DEVICE**

(30) Priority: 02.05.2014 JP 2014094910
(71) Applicant: Hitachi Zosen Corporation, Osaka-shi, Osaka 559-8559 (JP)
(72) Inventor: OGAWA Satoshi, Osaka 559-8559 (JP); KOBAYASHI Shimpei, Osaka 559-8559 (JP)
(74) Representative: Engelhardt, Harald
(86) International application number: PCT/JP2015/055503
(87) International publication number: WO 2015/166698

(57) **Abstract**

An electron beam sterilization method for a bottle cap is provided to sterilize a bottle cap (C) by dropping the bottle cap (C) sequentially along a sterilization path (15e) in the vertical direction while the bottle cap (C) is oriented such that the cap axis thereof passing through the center of an opening face is substantially horizontal, and applying an electron beam onto the bottle cap (C). The method has the following steps. First, in the sterilization path (15e), an ambient gas is sucked through a suction hole (25) formed along the vertical direction in a guide wall, thereby drawing the closed face of the bottle cap (C) toward the guide wall, and an electron beam is applied onto the opening face of the bottle cap (C). Next, in the sterilization path, an ambient gas is sucked through a suction hole formed along the vertical direction in a guide wall, thereby drawing the opening face of the bottle cap toward the guide wall, and an electron beam is applied onto the closed face of the bottle cap.

## Description

### TECHNICAL FIELD

The present invention relates to an electron beam sterilization method for a bottle cap and an electron beam sterilization device in which the bottle cap to be fitted on a mouth of a bottle is sterilized with electron beams.

### BACKGROUND

Patent Literature 1 discloses a technique of sterilizing a bottle cap in which the bottle cap is dropped into a cylindrical vacuum vessel so as to rotate about the horizontal axis passing through the cavity of the bottle cap, and at least three electron beam emitting means arranged around the vacuum vessel apply electron beams through respective windows to the bottle cap dropping.

### RELEVANT REFERENCES

### PATENT LITERATURE

Patent Literature 1: Japanese Patent No. 4380216

### SUMMARY

In Patent Literature 1, the bottle cap dropping while rotating about the horizontal axis thereof is oriented unstably due to rotation about the vertical axis along the direction of dropping, and therefore, it is necessary to arrange the three electron beam emitting means each covering a range of 120° around the vertical axis, thereby to apply electron beams to the bottle cap from three directions.

The electron beams emitted by the electron beam emitting units from the vacuum housing through the beam emitting windows may collide with air molecules and scatter to produce an ozone gas. The ozone gas may cause corrosion in the sterilization vessel Therefore, in a sterilization vessel containing an ambient gas (the air) instead of a vacuum, it is necessary to discharge the produced ozone gas and introduce a fresh ambient gas.

One object of the present invention is to provide an electron beam sterilization method for a bottle cap and an electron beam sterilization device in which the orientation of a dropping bottle cap is stabilized by sucking.or feeding of an ambient gas from or into a sterilization vessel, so as to uniformly sterilize the bottle cap with fewer electron beam emitting units.

An electron beam sterilization method for a bottle cap according to the first aspect includes: dropping a bottle cap, having a closed face and an opening face on opposite sides thereof, into a sterilization path in a vertical direction so as to orient the bottle cap such that a cap axis thereof passing through a center of the opening face is horizontal; sucking, in the sterilization path, an ambient gas through a suction hole formed along the vertical direction to draw one of the closed face and the opening face of the bottle cap, while applying an electron beam onto the other of the closed face and the opening face; and sucking, in the sterilization path, an ambient gas through a suction hole formed along the vertical direction to draw the other of the closed face and the opening face of the bottle cap, while applying an electron beam onto the one of the closed face and the opening face.

An electron beam sterilization method for a bottle cap according to the second aspect is configured as in the first aspect, wherein an ambient gas is sucked from or injected into a periphery around the bottle cap dropping in the sterilization path, in a substantially horizontal direction orthogonal to the cap axis so as to stabilize the orientation of the bottle cap.

An electron beam sterilization method for a bottle cap according to the third aspect is configured as in the first or second aspect, wherein in an inlet side of the sterilization path, an ambient gas is jetted downward onto the bottle cap so as to stabilize the orientation of the bottle cap and accelerate the drop of the bottle cap.

An electron beam sterilization device for a bottle cap according to the fourth aspect includes: a sterilization path in which a bottle cap is to be dropped in a vertical direction, the bottle cap having a closed face and an opening face on opposite sides thereof; a first sterilization section and a second sterilization section provided on the sterilization path; and a cap feeder provided on an inlet side of the sterilization path and configured to feed the bottle caps one by one so as to orient the bottle cap such that a cap axis thereof passing through a center of the opening face is substantially horizontal, wherein the first sterilization section includes a first electron beam emitting unit and a first orientation stabilizing unit, the first electron beam emitting unit being configured to emit an electron beam through a first emission window onto one of the closed face and the opening face of the bottle cap, the first orientation stabilizing unit being opposed to the first emission window and having a suction hole formed therein, the suction hole formed along the vertical direction and configured to draw the other of the closed face and the opening face, and wherein the second sterilization section includes a second electron beam emitting unit and a second orientation stabilizing unit, the second electron beam emitting unit being configured to emit an electron beam through a second emission window onto the other of the closed face and the opening face of the bottle cap, the second orientation stabilizing unit being opposed to the first emission window and having a suction hole formed therein, the suction hole formed along the vertical direction and configured to draw the one of the closed face and the opening face.

An electron beam sterilization device for a bottle cap according to the fifth aspect is configured as in the fourth aspect, wherein each of the first sterilization section and the second sterilization section is provided with a cap feeder on an inlet thereof.

An electron beam sterilization device for a bottle cap according to the sixth aspect is configured as in the fourth aspect, wherein the first sterilization section and the second sterilization section are provided sequentially on the sterilization path.

An electron beam sterilization device for a bottle cap according to the seventh aspect is configured as in the fourth to sixth aspect, wherein at least one of the first orientation stabilizing unit and the second orientation stabilizing unit is provided with a suction/injection hole for sucking or injecting an ambient gas from or into a periphery around the bottle cap dropping in the sterilization path, in a substantially horizontal direction orthogonal to the cap axis so as to stabilize the orientation of the bottle cap.

### ADVANTAGES

In the first aspect, a bottle cap is dropped in the sterilization path in the vertical direction so as to be oriented such that the cap axis thereof is substantially horizontal. One of the closed face and the opening face of the bottle cap is drawn toward the suction hole to stabilize the orientation of the bottle cap, and an electron beam can be securely applied onto the other of the closed face and the opening face. Accordingly, the entire surface of the bottle cap can be uniformly sterilized with only two electron beam emitting units for applying electron beams onto the closed face and the opening face, respectively. The electron beams colliding with molecules of an ambient gas and are scattered and applied onto the outer peripheral surface of the bottle cap, and therefore, the outer peripheral surface of the bottle cap is sterilized satisfactorily.

In the second aspect, an ambient gas is further injected into or sucked from a periphery around the bottle cap dropping in the sterilization path so as to further stabilize the orientation thereof, and therefore, stable sterilization can be achieved with the electron beams.

In the third aspect, an ambient gas is jetted toward the bottle gap dropping in the inlet side so as to accelerate the bottle cap downward and stabilize the orientation thereof, thus increasing the processing speed

In the fourth aspect, the first and second sterilization sections are configured such that the air is sucked from a suction hole to stabilize the dropping bottle cap in the horizontal direction, and an electron beam is applied from the direction opposite to the sucking direction for sterilization. Thus, the opening face and the closed face of the bottle cap can be sterilized securely, and the lateral periphery of the bottle cap can be sterilized satisfactorily by scattering electron beams caused by collision with the air molecules.

In the seventh aspect, the orientation stabilizing unit is provided with a suction/injection hole for sucking or injecting an ambient gas from or into a periphery around the bottle cap, and therefore, the orientation of the dropping bottle cap can be further stabilized

### BRIEF DESCRIPTION OF THE DRAWINGS

Fig. 1 is a sectional view showing the entirety of an embodiment of an electron beam sterilization device for a bottle cap according to the present invention as viewed from the front.
Fig. 2 is a front view showing an arrangement of electron beam emitting units in the electron beam sterilization device.
Fig. 3 is a sectional view along the line A-A in Fig. 1.
Fig. 4 is a sectional view along the line B-B in Fig. 1.
Fig. 5 is a sectional side view of a first sterilization section.
Fig. 6 is a sectional view along the line D-D in Fig. 5.
Fig. 7 is a sectional side view of a second sterilization section.
Fig. 8 is a sectional view along the line E-E in Fig. 7.
Fig. 9 is a sectional plan view of the first sterilization section of another embodiment.
Fig. 10 is a sectional plan view of the first sterilization section of still another embodiment using a nozzle-shaped electron beam emitting unit.

### DESCRIPTION OF EXAMPLE EMBODIMENTS

Embodiments of the present invention will be hereinafter described with reference to the drawings. In an electron beam sterilization device for a bottle cap according to the present invention, a bottle cap C including a closed face Co formed in the top thereof and an opening face Ci formed in the bottom thereof may be dropped in an ambient gas such as the air along the vertical direction in such an orientation that the cap axis passing through the center of the opening face Ci may be substantially horizontal, and electron beams may be applied to the dropping bottle cap for sterilization.

### (Entire Structure)

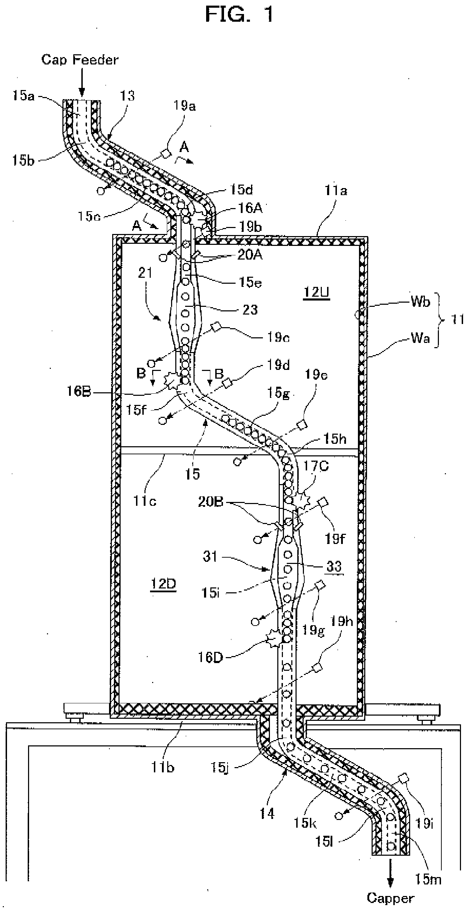

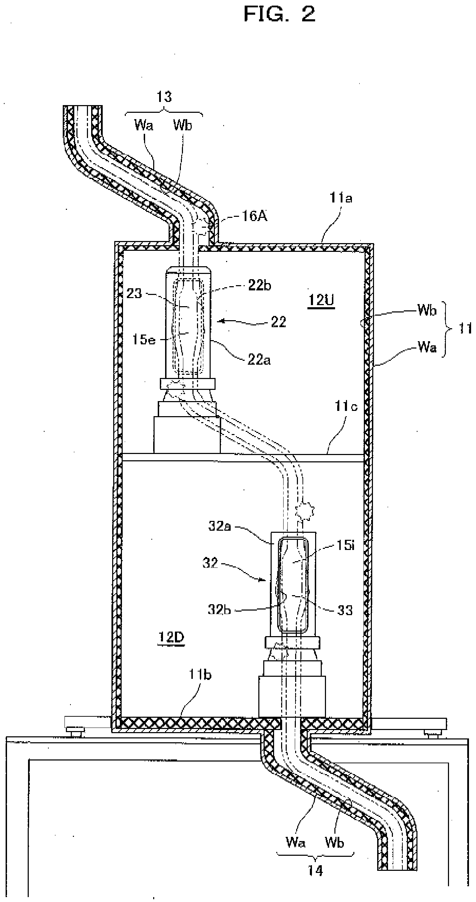

As shown in Figs. 1 and 2, there maybe installed a screening vessel 11 for sterilizing bottle caps made of a structural wall Wa and a screening wall Wb for screening out X rays. The screening vessel 11 may be partitioned by an intermediate frame 11c into an upper chamber 12U containing a first sterilization section 21 and a lower chamber 12D containing a second sterilization section 31. The upper chamber 12U may have a through-hole formed in the ceiling surface 11a thereof. This through-hole may be connected with an inlet screening pipe 13 constituted by the structural wall Wa and the screening wall Wb. The lower chamber 12D may have a through-hole formed in the floor surface 11b thereof. This through-hole may be connected with an outlet screening pipe 14 constituted by the structural wall Wa and the screening wall Wb. A sterilization path 15 may extend from the inlet screening pipe 13 through the screening vessel 11 to the outlet screening pipe 14. The bottle caps C may be dropped along the sterilization path 15 at a rate of 300 to 600 bottle caps per minute, for example. The electron beams may be applied onto the closed face Co and the opening face Ci of a bottle caps C to sterilize the entire bottle cap C.

### (Sterilization Path)

The sterilization path 15 may include, e.g., a plurality of dropping portions 15a, 15e, 15i, and 15m extending along the vertical direction, a plurality of waiting portions 15c, 15g, and 15k extending obliquely at a predetermined angle of, e.g., 45 to 70° (this angle is about 60° in the drawing) with respect to the vertical line, and bent portions 15b, 15d, 15f, 15h, 15j, and 15l The dropping portions 15a, 15e, 15i, and 15m and the waiting portions 15c, 15g, and 15k may be connected together via the bent portions 15b, 15d, 15f, 15h,15j, and 15l.

That is, the inlet screening pipe 13 may encircle the inlet dropping portion 15a, the inlet bent portion 15b, the first waiting portion 15c, and the first bent portion 15d of the sterilization path 15 connected in this order. The inlet screening pipe 13 may be bent in accordance with the inlet dropping portion 15a, the inlet bent portion 15b, the first waiting portion 15c, and the first bent portion 15d so as to prevent leakage of X rays out of the screening vessel 11. The first bent portion 15d may be provided with a first feeding star wheel (first cap feeder) 16A.

The upper chamber 12U may contain the first dropping portion 15e, the second bent portion 15f, and the second waiting portion 15g connected in this order. The first dropping portion 15e may be provided with the first sterilization section 21, and the second bent portion 15f may be provided with a first receiving start wheel 16B. The lower chamber 12D may contain the third bent portion 15h and the second dropping portion 15i connected in this order. The second dropping portion 15i may be provided with a second feeding star wheel (second cap feeder) 16C on the inlet side, the second sterilization section 31 in an intermediate portion, and a second receiving star wheel 16D on the outlet side.

Further, the outlet screening pipe 14 may encircle the fourth bent portion 15j, the outlet waiting portion 15k, the outlet bent portion 151, and the outlet dropping portion 15m connected in this order. The outlet screening pipe 14 may be bent in accordance with the fourth bent portion 15j, the outlet waiting portion 15k, the outlet bent portion 15l, and the outlet dropping portion 15m, so as to prevent leakage of X rays out of the screening vessel 11.

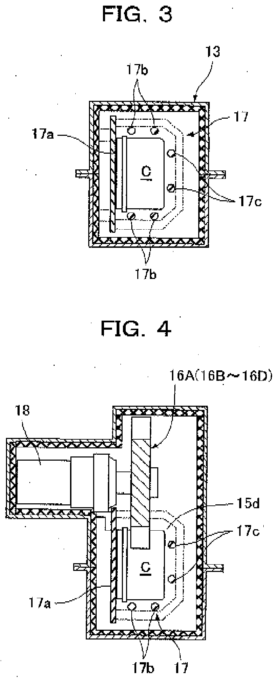

As shown in Fig. 3, the sterilization path 15 except the first and second sterilization sections 21, 31 may be provided with a cap guide 17 for guiding a bottle cap in such an orientation that the cap axis thereof may be substantially horizontal The cap guide 17 may include a guide plate 17a for guiding the opening face Ci of the bottle cap C, one or more side guide rods 17b for guiding the left and right side faces of the bottle cap C, one or more closed face guide rod 17c for guiding the closed face Co of the bottle cap C, and support members 17d for these guide rods. As shown in Fig, 4, the first and second feeding star wheels 16A, 16C and the first and second receiving star wheels 16B, 16D may be each rotated intermittently by a step motor 18 through one-eighth of a revolution to sequentially feed the bottle caps forward

In Fig. 1, the reference signs 19a to 19i denote non-contact sensors such as photoelectric cap sensors arranged at predetermined locations in the sterilization path 15 and configured to detect the bottle caps C. Based on detection signals from the cap sensors 19a to 19i, the first and second feeding star wheels 16A, 16C and the first and second receiving star wheels 16B, 16D may be controlled The reference signs 20A, 20B denote first and second assist nozzles for assisting the drop of the bottle caps C. These assist nozzles may be arranged below the first and second feeding star wheels 16A, 16C. The first and second assist nozzles 20A, 20B may apply a jet of the ambient gas downward onto the dropping bottle caps C. The ambient gas jetted from the first and second assist nozzles 20A, 20B may stabilize the orientation of the bottle caps C fed from the first and second feeding star wheels 16A, 16C and accelerate the drop of the bottle caps C.

### (First Sterilization Section and Second Sterilization Section)

The first sterilization section 21 may be provided with a first electron beam emitting unit 22, and the second sterilization section 31 may be provided with a second electron beam emitting unit 32. The first and second electron beam emitting units 22, 32 may include vacuum vessels 22a, 32a containing a filament and electrodes and having emission windows 22b, 32b formed thereon. The emission windows 22b, 32b may face a first sterilization chamber 23 and a second sterilization chamber 33 provided in the first and second dropping portions 15i, 15e of the sterilization path 15, respectively. The first sterilization chamber 23 and the second sterilization chamber 33 may have a larger width and depth than the first and second dropping portions 15i, 15e, respectively. The first sterilization chamber 23 may be provided with first and second orientation stabilizing units 24, and the second sterilization chamber 33 may be provided with a second orientation stabilizing unit 34, so as to stabilize the orientation of the bottle caps C in the first and second dropping portions 15i, 15e.

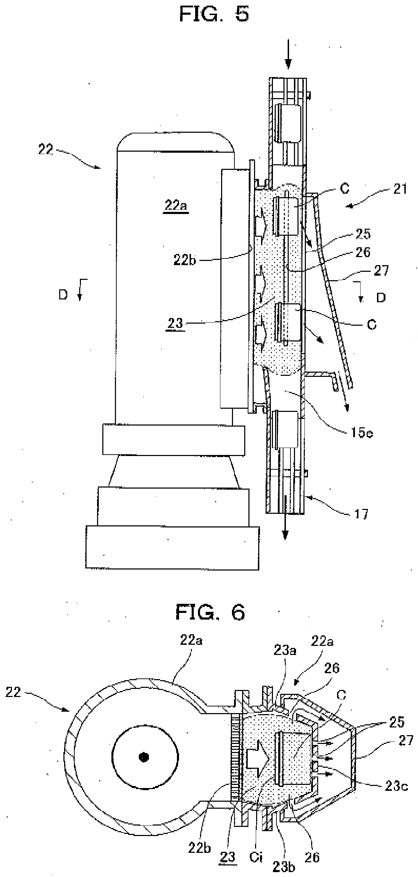

As shown in Fig. 5 and the sectional plan view of Fig. 6, a bottle cap C may drop in the first sterilization section 21 with the opening face Ci thereof facing the emission window 22b of the first electron beam emitting unit 22. The first sterilization chamber 23 may have, e.g., an isosceles trapezoid shape in a sectional plan view. More specifically, the first sterilization chamber 23 may have an opening face on the emission window 22b side, a guide wall 23c opposed to the emission window 22b, and side walls 23a, 23b on the left and right sides. The opening face may correspond to the lower base of the trapezoid, the guide wall 23c may correspond to the upper base of the trapezoid, and the side walls 23a, 23b may correspond to the two legs of the trapezoid converging with depth (toward the closed face Co). The first orientation stabilizing unit 24 may include one or more suction slits (suction holes) 25 formed in the guide wall 23c along the vertical (dropping) direction (the drawing shows three suction slits), and one or more side slits (suction/injection holes) 26 formed in each of the left and right side walls 23a, 23b along the vertical (dropping) direction (the drawing shows one side slit in each side wall). Further, on the back side of the first sterilization chamber 23, there may be provided a suction cover 27 covering the suction slits 25 and the left and right side slits 26. The suction cover 27 may be connected with a suction hose which may be connected to a suction pump (not shown). The suction cover 27 may cause the ambient gas (e.g., the air) in the first sterilization chamber 23 to be sucked obliquely downward through the suction slit 25 and the left and right side slits 26.

The second bent portion 15f may be provided with the first receiving star wheel 16B for receiving the bottle caps dropping from the first sterilization chamber 23. The first receiving star wheel 16B may sequentially feed the bottle caps C toward the second waiting portion 15g.

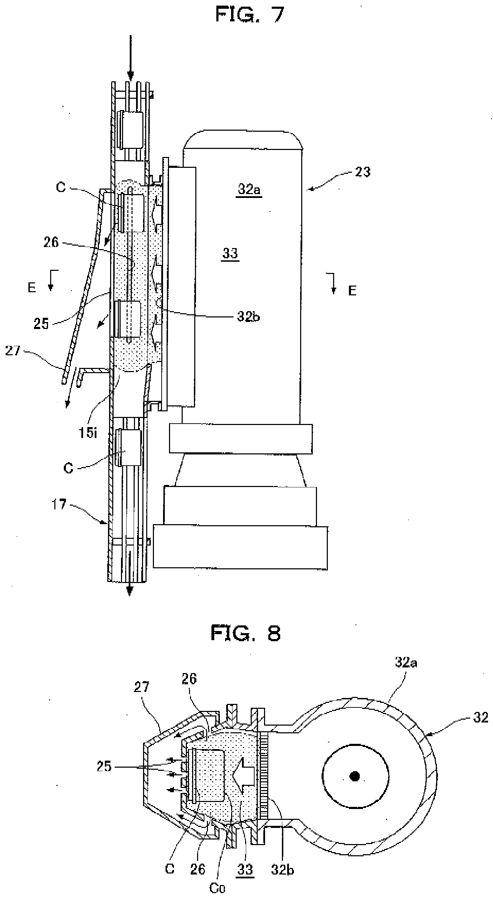

Further, as shown in Fig. 7 and the sectional plan view of Fig. 8, the second sterilization section 31 may include the second sterilization chamber 33 in which the closed face Co of a bottle cap C may face an emission window 32b of the second electron beam emitting unit 32. The second sterilization chamber 33 may have, e.g., an isosceles trapezoid shape in a sectional plan view. More specifically, the second sterilization chamber 33 may have an opening face on the emission window 32b side, a guide wall 33c opposed to the emission window 32b, and side walls 33a, 33b on the left and right sides. The opening face may correspond to the lower base of the trapezoid, the guide wall 33c may correspond to the upper base of the trapezoid, and the side walls 33a, 33b may correspond to the two legs of the trapezoid converging with depth (toward the opening face Ci). The second orientation stabilizing unit 34 may include one or more suction slits (suction holes) 35 formed in the guide wall 33c along the vertical (dropping) direction (the drawing shows three suction slits), and one or more side slits (suction/injection holes) 36 formed in each of the left and right side walls 33a, 33b along the vertical (dropping) direction (the drawing shows one side slit in each side wall). Further, on the front side of the second sterilization chamber 33, there maybe provided a suction cover 37 covering the suction slits 35 and the left and right side slits 36. The suction cover 37 may be connected with a suction hose which may be connected to a suction pump (not shown). The ambient gas (e.g., the air for this embodiment) in the second sterilization chamber 33 maybe sucked obliquely downward through the suction slit 35 and the left and right side slits 36.

Below the second sterilization chamber 33 in the second dropping portion 15i, there may be provided the second receiving star wheel 16D for receiving the bottle caps C. The second receiving star wheel 16D may sequentially feed the bottle caps C through the fourth bent portion 51j, the outlet waiting portion 15k, and the outlet bent portion 151 to the outlet dropping portion 15m.

### (Sterilization Operation)

In the above arrangement, a cap feeder (not shown) may sequentially feed the bottle caps C into the inlet dropping portion 15a of the sterilization path 15. At this time, the bottle caps C may be oriented such that the cap axis thereof is horizontal and the opening face Ci thereof faces the front. The dropped bottle caps C may be retained by the first feeding star wheel 16A in a range from the inlet bent portion 51b through the first waiting portion 51c to the first bent portion 15d. The bottle caps C may then be fed into the first dropping portion 15e one by one by the rotation of the first feeding star wheel 16A. In the first sterilization section 21, the electron beams emitted through the emission window 22b of the first electron beam emitting unit 22 may be applied to the opening face Ci side of a bottle cap C so as to sterilize the opening face Ci and the outer peripheral surface. In the first sterilization section 21, the first orientation stabilizing unit 24 may cause the ambient gas in the first sterilization chamber 23 to be sucked from around the bottle cap C through the suction slits 25 and the left and right side slits 26. Thus, the closed face Co of the bottle cap C may be drawn toward the guide wall 23c, and the bottle cap C may drop stably without rotating about the vertical axis thereof. Accordingly, the electron beams can be effectively applied to the inside of the opening face Ci and the outer periphery of the bottle cap C, thus achieving stable sterilization.

The bottle caps C dropped from the first dropping portion 15e to the second bent portion 15f may be restrained from dropping and retained by the first receiving star wheel 16B, and sequentially fed to the second waiting portion 15g.

The second feeding star wheel 16C may sequentially feed the bottle caps C retained in the second waiting portion 15g and the third bent portion 15h into the second dropping portion 15i. In the second sterilization section 31 of the second dropping portion 15i, the electron beams emitted through the emission window 32b of the second electron beam emitting unit 32 may be applied to a bottle cap C. Simultaneously, the second orientation stabilizing unit 34 may cause the ambient gas in the second sterilization chamber 33 to be sucked from around the bottle cap C through the suction slits 35 and the side slits (suction/injection holes) 36. Thus, the opening face Ci of the bottle cap C may be drawn toward the guide wall 33c, and the bottle cap C may drop stably without rotating about the vertical axis thereof. Accordingly, the electron beams can be effectively applied to the closed face Co and the outer peripheral surface of the cap C, thus achieving stable sterilization.

The bottle caps C dropped from the second dropping portion 15i to the fourth bent portion 15j may be discharged through the outlet waiting portion 15k, the outlet bent portion 151, and the outlet dropping portion 15m, and then fed to a capper for fitting the bottle caps onto the bottles filled with contents.

In the above embodiment, the first and second orientation stabilizing units 24, 34 of the first and second sterilization sections 21, 31 may respectively cause the ambient gas in the first sterilization chamber 23 and the second sterilization chamber 33 to be sucked through the suction slits 25, 35 so as to draw a bottle cap C toward the guide walls 23c, 33c. Thus, the bottle cap C may be prevented from rotating about the vertical axis along the dropping direction, and thus the bottle cap C can drop with the orientation thereof stabilized Further, the ambient gas around the bottle cap C may be sucked through the left and right side slits 26, 36 so as to further stabilize the orientation of the bottle cap C. Accordingly, the electron beams emitted through the emission windows 22b, 32b of the first and second electron beam emitting units 22, 32 may be satisfactorily applied to the opening face Ci and the closed face Co of the bottle cap C, and the electron beams colliding with the molecules of the ambient gas and scattered may satisfactorily sterilize the outer peripheral surface of the bottle cap C.

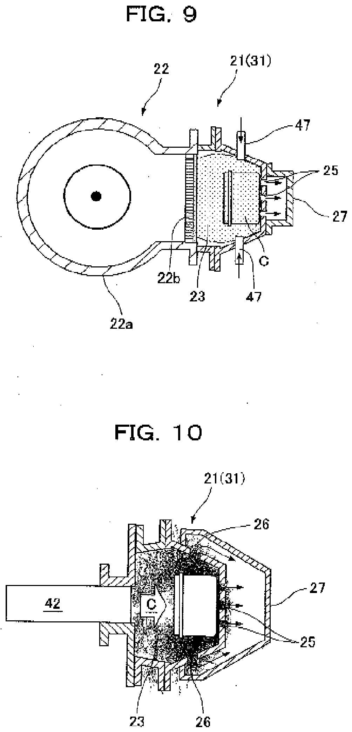

As shown in Fig. 9, the side slits 26, 36 may be replaced with injection nozzles 47 or injection slits for injecting the ambient gas. The ambient gas may be injected uniformly downward onto the left and right sides of a bottle cap C by the injection nozzles 47 or the injection slits, so as to further stabilize the orientation of the bottle cap C.

In the above embodiment, the first sterilization section 21 and the second sterilization section 31 may be provided in the first and second dropping portions 15e, 15i, respectively. It may also be possible to provide the first sterilization section 21 and the second sterilization section 31 in one dropping portion in a vertical sequential arrangement.

Further, in the above embodiment, the electron beam emitting units 22, 32 may have such a large size as to sterilize the outer surface of a bottle. The electron beam emitting units 22, 32 may be replaced with nozzle-type electron beam emitting units 42 that can be inserted into a mouth of a bottle, as shown in Fig. 10. In this case, each sterilization section can be provided with two or more nozzle-type electron beam emitting units 42 in a vertical sequential arrangement. Fig. 10 simulates scattering of the electron beams emitted from the electron beam emitting unit 42 and colliding with the air molecules by the Monte Carlo method

## Claims

1. An electron beam sterilization method for a bottle cap, comprising:
dropping a bottle cap, having a closed face and an opening face on opposite sides thereof, into a sterilization path in a vertical direction so as to orient the bottle cap such that a cap axis thereof passing through a center of the opening face is horizontal;
sucking, in the sterilization path, an ambient gas through a suction hole formed along the vertical direction to draw one of the closed face and the opening face of the bottle cap, while applying an electron beam onto the other of the closed face and the opening face; and
sucking, in the sterilization path, an ambient gas through a suction hole formed along the vertical direction to draw the other of the closed face and the opening face of the bottle cap, while applying an electron beam onto the one of the closed face and the opening face.

2. The electron beam sterilization method for a bottle cap according to claim 1, wherein an ambient gas is sucked from or injected into a periphery around the bottle cap dropping in the sterilization path, in a substantially horizontal direction orthogonal to the cap axis so as to stabilize the orientation of the bottle cap.

3. The electron beam sterilization method for a bottle cap according to claim 1 or 2, wherein in an inlet side of the sterilization path, an ambient gas is jetted downward onto the bottle cap so as to stabilize the orientation of the bottle cap and accelerate the drop of the bottle cap.

4. An electron beam sterilization device for a bottle cap, comprising:
a sterilization path in which a bottle cap is to be dropped in a vertical direction, the bottle cap having a closed face and an opening face on opposite sides thereof;
a first sterilization section and a second sterilization section provided on the sterilization path; and
a cap feeder provided on an inlet side of the sterilization path and configured to feed the bottle caps one by one so as to orient the bottle cap such that a cap axis thereof passing through a center of the opening face is substantially horizontal,
wherein the first sterilization section includes a first electron beam emitting unit and a first orientation stabilizing unit, the first electron beam emitting unit being configured to emit an electron beam through a first emission window onto one of the closed face and the opening face of the bottle cap, the first orientation stabilizing unit being opposed to the first emission window and having a suction hole formed therein, the suction hole formed along the vertical direction and configured to draw the other of the closed face and the opening face, and
wherein the second sterilization section includes a second electron beam emitting unit and a second orientation stabilizing unit, the second electron beam emitting unit being configured to emit an electron beam through a second emission window onto the other of the closed face and the opening face of the bottle cap, the second orientation stabilizing unit being opposed to the first emission window and having a suction hole formed therein, the suction hole formed along the vertical direction and configured to draw the one of the closed face and the opening face.

5. The electron beam sterilization device for a bottle cap according to claim 4, wherein each of the first sterilization section and the second sterilization section is provided with a cap feeder on an inlet thereof.

6. The electron beam sterilization device for a bottle cap according to claim 4, wherein the first sterilization section and the second sterilization section are provided sequentially on the sterilization path.

7. The electron beam sterilization device for a bottle cap according to any one of claims 4 to 6, wherein at least one of the first orientation stabilizing unit and the second orientation stabilizing unit is provided with a suction/injection hole for sucking or injecting an ambient gas from or into a periphery around the bottle cap dropping in the sterilization path, in a substantially horizontal direction orthogonal to the cap axis so as to stabilize the orientation of the bottle cap.
